# EUROPÄISCHE PATENTANMELDUNG

(11) **EP 3 679 953 A2**
(43) Veröffentlichungstag der Anmeldung: **15.07.2020**
(21) Anmeldenummer: 20150416.4
(22) Anmeldetag: 07.01.2020
(51) Int. Cl.: A61L 2/26

(54) **SIEBKORB UND DECKEL HIERFÜR**

(30) Priorität: 09.01.2019 DE 102019100437
(71) Anmelder: OLYMPUS Winter & Ibe GmbH, 22045 Hamburg (DE)
(72) Erfinder: Wolter, Michael, 22307 Hamburg (DE); Jarms, Ann-Kathrin, 21033 Hamburg (DE)
(74) Vertreter: Glawe, Delfs, Moll

(57) **Zusammenfassung**

Die Erfindung betrifft einen Siebkorb (1) für die Aufbereitung von medizinischen Instrumenten mit im Wesentlichen rechteckiger Grundfläche (2) und offener Oberseite (3), umfassend einen ersten und einen zweiten Behälterteil (4', 4"), die an jeweils einer Stirnseite offen sind und eine siebartige Bewandung (5) aufweisen, wobei der erste Behälterteil (4') zum Einschieben in den zweiten Behälterteil (4") ausgebildet ist und wenigstens ein durch geeignete Durchstecköffnungen (20) hindurchsteckbaren Sicherungsstift (10) zur wahlweisen Sicherung der ineinandergeschobenen Behälterteile (4', 4") in wenigstens zwei unterschiedlichen Positionen zueinander vorgesehen ist.

Die Erfindung betrifft weiterhin einen Deckel für einen - vorzugsweise erfindungsgemäßen - Siebkorb, umfassend einen ersten und einen zweiten Deckelteil, die in wenigstens zwei verschiedenen Position zueinander anordnenbar sind und wenigstens ein durch geeignete Durchstecköffnungen hindurchsteckbaren Sicherungsstift (10) zur wahlweisen Sicherung der Deckelteile in wenigstens zwei unterschiedlichen Positionen zueinander vorgesehen ist.

## Beschreibung

Die Erfindung betrifft einen Siebkorb für die Aufbereitung von medizinischen Instrumenten sowie einen Deckel für einen entsprechenden Siebkorb.

Medizinische und insbesondere chirurgische Instrumente müssen nach ihrer Verwendung grundsätzlich aufbereitet werden, bevor sie wiederverwendet werden können. Zu der Aufbereitung gehören Reinigung, Desinfektion und evtl. auch Sterilisation der Instrumente. Die Reinigung und Desinfektion kann in dafür ausgebildeten Reinigungsdesinfektionsgeräten durchgeführt werden, bevor die gereinigten und desinfizierten Instrumente bei Bedarf in einem Sterilisator sterilisiert werden können.

Es ist bekannt, die maschinell aufzubereitenden medizinischen Instrumente zunächst einzeln oder in Gruppen in als Siebkörbe ausgebildete Reinigungskörbe - auch Trays genannt - eingelegt werden, bevor der Siebkorb mit den darin enthaltenen Instrumenten in ein Reinigungsdesinfektionsgerät eingebracht wird, bevor er nach erfolgter Reinigung und Desinfektion bei Bedarf einem Sterilisator zugeführt wird.

Der Siebkorb dient dabei als Schutz vor Beschädigungen und Rekontamination der darin enthalten Instrumente. Dabei sind die Siebkörbe aus dem Stand der Technik starre und unflexible Gestelle. Dabei gibt es für die verschiedenen Anwendungsfälle Gestelle in unterschiedlichen Formen und Größen, die jeweils vorgehalten werden müssen und somit teils erheblichen Lagerraum beanspruchen.

Aufgabe der vorliegenden Erfindung ist es, einen Siebkorb für die Aufbereitung von medizinischen Instrumenten sowie einen Deckel dafür zu schaffen, bei dem die Nachteile aus dem Stand der Technik nicht mehr oder nur noch in vermindertem Umfang auftreten.

Gelöst wird diese Aufgabe durch einen Siebkorb gemäß dem Hauptanspruch sowie einen Deckel gemäß dem nebengeordneten Anspruch. Vorteilhafte Weiterbildungen sind Gegenstand der abhängigen Ansprüche.

Demnach betrifft die Erfindung einen Siebkorb für die Aufbereitung von medizinischen Instrumenten mit im Wesentlichen rechteckiger Grundfläche und offener Oberseite, umfassend einen ersten und einen zweiten Behälterteil, die an jeweils einer Stirnseite offen sind und eine siebartige Bewandung aufweisen, wobei der erste Behälterteil zum Einschieben in den zweiten Behälterteil ausgebildet ist und wenigstens ein durch geeignete Durchstecköffnungen hindurchsteckbaren Sicherungsstift zur wahlweisen Sicherung der ineinandergeschobenen Behälterteile in wenigstens zwei unterschiedlichen Positionen zueinander vorgesehen ist.

Die Erfindung betrifft weiterhin einen Deckel für einen - vorzugsweise erfindungsgemäßen - Siebkorb, umfassend einen ersten und einen zweiten Deckelteil, die in wenigstens zwei verschiedenen Position zueinander anordnenbar sind und wenigstens ein durch geeignete Durchstecköffnungen hindurchsteckbaren Sicherungsstift zur wahlweisen Sicherung der Deckelteile in wenigstens zwei unterschiedlichen Positionen zueinander vorgesehen ist.

Die Erfindung hat erkannt, dass die für einen Siebkorb für die Aufbereitung von medizinischen Instrumenten erforderliche Tragkraft auch durch zwei lösbar miteinander verbindbare Behälterteile erreicht werden kann, die es darüber hinaus gestatten, den Siebkorb größenveränderlich auszugestalten. Dabei können die beiden Behälterteile so ineinandergeschoben werden, dass ihre jeweilige Bewandung sich zu einer Bewandung eines Siebkorbs mit rechteckiger Grundfläche und offener Oberseite zusammenfügt. Zum wahlweisen Fixieren der beiden Behälterteile zueinander ist wenigstens ein Sicherungsstift vorgesehen, der, wenn in dafür vorgesehene Durchstecköffnungen eingeführt, die ineinandergeschobenen Behälterteile gegeneinander sichert. Dabei ist vorgesehen, entsprechende Sicherungsstifte und/oder Durchstecköffnungen vorzusehen, um die beiden Behälterteile in wenigstens zwei unterschiedlichen Positionen zueinander sichern zu können, womit letztendlich die Größeneinstellbarkeit des Siebkorbs gewährleistet ist.

Der wenigstens eine Sicherungsstift kann dabei unlösbar an einem Behälterteil als aus dessen Bewandung hervorstehender Schaft mit Rastelement am Kopfende ausgebildet sein, wobei der andere Behälterteil dann wenigstens zwei Durchstecköffnungen aufweist, in den der wenigstens eine Sicherungsstift eingeführt werden kann. Der wenigstens eine Sicherungsstift wird dann beim Einschieben des anderen Behälterteils in den einen Behälterteil in die Durchstecköffnung eingeführt und sichert die beiden Behälterteile dann zueinander. Die Anzahl der Durchstecköffnungen ist dabei grundsätzlich größer als die Anzahl der Sicherungsstifte, in der Regel wenigstens doppelt so groß. Der Sicherungsstift kann einstückig mit der Bewandung ausgestaltet oder unlösbar (bspw. durch Schweißen oder Kleben) mit dieser verbunden sein.

Alternativ dazu ist es möglich, dass der wenigstens eine Sicherungsstift einen Schaft mit einem Halteelement am Fußende und einem Rastelement am Kopfende zum Durchstecken durch fluchtende Durchstecköffnungen in beiden Behälterteilen ausgebildet ist. In diesem Fall ist der wenigstens eines Sicherungsstift also ein separates Bauteil, welches in geeignete Durchstecköffnungen in den Behälterteilen geführt werden kann, um diese zueinander zu sichern. Auch hier ist eine geeignete Anzahl an Durchstecköffnungen in wenigstens einem Behälterteil vorgesehen, die in der Regel größer, häufig wenigstens doppelt so groß ist wie die Anzahl der Sicherungsstifte.

Im Falle von einem oder mehreren separaten Sicherungsstiften kann eine Führung zwischen den beiden Behälterteilen vorgesehen sein, welche eine relative Bewegung der ineinandergeschobenen Behälterteile senkrecht zur offenen Oberseite verhindert. In diesem Fall ist es ausreichend, wenn der wenigstens eine Sicherungsstift eine Bewegung entlang der Führung unterbinden kann. Insbesondere muss der wenigstens eine Sicherungsstift keinen Kräften in Richtung senkrecht zur offenen Oberseite standhalten, da diese von der Führung aufgenommen werden.

Es ist bevorzugt, wenn wenigstens ein Behälterteil wenigstens ein Positionierungselemente aufweist, welches ein Sichern der beiden Behälterteile nur in solchen Positionen zulässt, in denen die Durchlöcherung der Bewandung der beiden Behälterteil im Überlappungsbereich der beiden Behälterteile wenigstens weitestgehend totraumfrei ist. Werden die beiden Behälterteile erfindungsgemäß durch den wenigstens einen Sicherungsstift miteinander verbunden, ergibt sich zwangsläufig ein Bereich, in dem die Bewandung der beiden Behälterteile überlappt. Je nach Ausgestaltung der Bewandung und insbesondere deren siebartiger Durchlöcherung besteht das Risiko, dass die einzelnen Öffnungen der Durchlöcherung der beiden Behälterteile nicht miteinander fluchten und sich so Bereiche bilden, in denen sich Reinigungsmittel oder Verschmutzungen ansammeln können. Sofern die Bildung von solchen Toträumen nicht bereits durch Anordnung der Durchstecköffnungen in den beiden Behälterteilen wirksam verhindert wird, können zusätzliche Positionierungselemente an wenigstens einem der Behälterteile vorgesehen sein, welche Anordnungen der beiden Behälterteile zueinander gewährleiten, in denen sich keine oder nur sehr wenig Toträume bilden.

Es ist bevorzugt, wenn das Rastelement des wenigstens einen Sicherungsstiftes werkzeugfrei lösbar ist. In anderen Worten sollen die beiden Behälterteile also bei Bedarf voneinander gelöst werden können (bspw., um zur Bildung eines Siebkorbs in einer anderen Größe wieder zusammengefügt werden zu können), ohne dass es hierfür Werkzeug erfordert. Die Werkzeugfreiheit erleichtert die Verwendung des Siebkorbs im medizinischen Alltag. Eine Möglichkeit zur Gewährleistung der Werkzeugfreiheit besteht in einer Ausgestaltung der Rastelemente derart, dass sich diese bei einer Losbrechkraft, die größer ist als die auf die Rastelemente bei ordnungsgemäßer Nutzung des Siebkorbs zu erwartenden Kräfte, beschädigungsfrei lösen.

Die Durchstecköffnungen können gesondert von einer siebartigen Durchlöcherung der Bewandung oder Bestandteil der Durchlöcherung sein. Je nach Ausgestaltung der Bewandung können die Durchstecklöcher mit den Sieböffnungen identisch sein, sodass keine separaten Durchstecklöcher vorgesehen werden müssen. Dabei ist regelmäßig eine solche Vielzahl an als Durchstecklöchern nutzbare Öffnungen vorhanden, dass eine besonders hohe Größenvariabilität des Siebkorbs erreicht wird. Es ist aber auch möglich, dass die Durchstecklöcher getrennt von der eigentlichen siebartigen Durchlöcherung gebildet sind, bspw. um besser an die Ausgestaltung und Dimensionierung der Sicherungsstifte angepasst zu sein.

Es ist bevorzugt, wenn wenigstens zwei, weiter vorzugsweise wenigstens vier Sicherungsstifte vorgesehen sind. Eine entsprechende Anzahl an Sicherungsstiften hat sich einerseits als ausreichend zur Gewährleistung der Stabilität des Siebkorbs, andererseits als einfach handhabbar erwiesen.

Die Behälterteile können wenigstens teilweise aus Lochblech, Drahtgeflecht oder geschweißtem Drahtgitter sein. Entsprechende Ausgestaltungen können die gewünschte Siebfunktion gewährleisten.

Die Behälterteile und/oder der wenigstens eine Sicherungsstift sind bevorzugt aus Edelstahl.

Es ist selbstverständlich möglich, auf der Innenseite des Siebkorbs bzw. der beiden Behälterteile Halter für aufzubereitende Instrumente anzuordnen. Die Halter können vorzugsweise lösbar an der Bewandung der Behälterteile befestigt sein, um eine flexible Ausstattung des Siebkorbs mit Halteelementen zu ermöglichen.

Der erfindungsgemäße Deckel ist analog zu dem beschriebenen Siebkorb ausgestaltet, d.h. er umfasst zwei Deckelteile, die sich in wenigstens zwei unterschiedlichen Positionen zueinander mit Hilfe eines Sicherungsstiftes sichern lassen, um so bspw. zu einem größenverstellbaren Siebkorb einen entsprechend anpassbaren Deckel bereitzustellen. Aufgrund der augenfälligen Analogie zum erfindungsgemäßen Siebkorb wird zur näheren Erläuterung des erfindungsgemäßen Deckels auf die vorstehenden Ausführungen verwiesen.

Die Erfindung wird nun anhand einer bevorzugten Ausführungsform unter Bezugnahme auf die beigefügten Zeichnungen beispielhaft beschrieben. Es zeigen:
- Figur 1a-d:: ein erstes Ausführungsbeispiel eines erfindungsgemäßen Siebkorbs; und
- Figur 2a-g:: ein zweites Ausführungsbeispiel eines erfindungsgemäßen Siebkorbs.

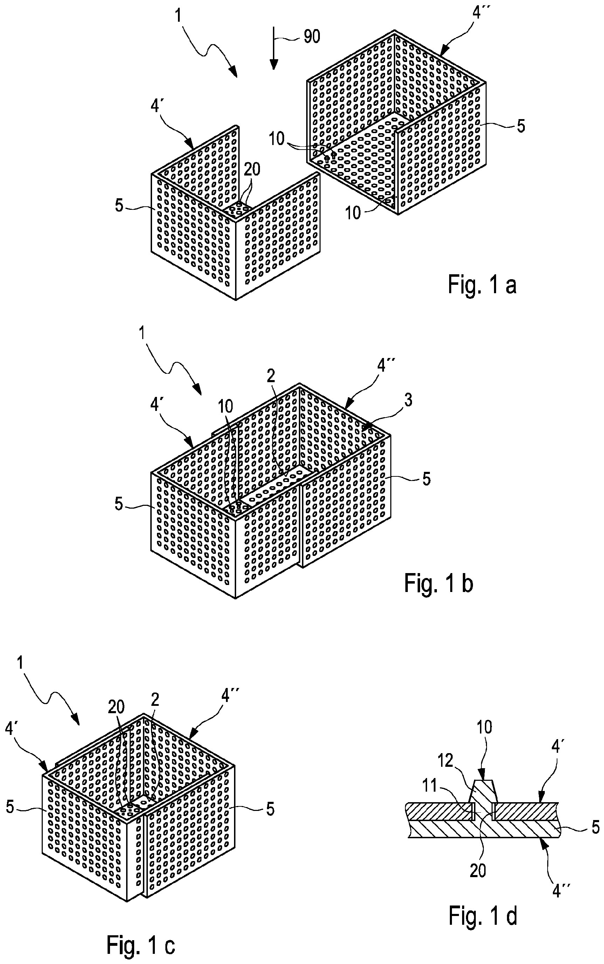

In Figur 1 ist ein erstes Ausführungsbeispiel eines erfindungsgemäßen Siebkorbs 1 für die Aufbereitung von medizinischen Instrumenten dargestellt. Dabei zeigt Figur 1a die beiden Behälterteile 4', 4" des Siebkorbs 1 separiert, während in Figuren 1a und 1b die beiden Behälterteile 4', 4" in unterschiedlichen Positionen zueinander ineinandergeschoben und gesichert sind. Figur 1d zeigt ein Detail der Sicherung der beiden Behälterteile 4', 4".

Der Siebkorb 1 für die Aufbereitung von medizinischen Instrumenten weist im ordnungsgemäß zusammengesetzten Zustand der beiden Behälterteile 4', 4'' eine rechteckige Grundfläche 2 und eine offene Oberseite 3 auf. Die beiden Behälterteile 4', 4" - der erste Behälterteil 4' und der zweite Behälterteil 4" - weisen dabei eine siebartige Bewandung 5 aus Edelstahl-Lochblech auf, und sind an jeweils einer Seite offen. Der erste Behälterteil 4' ist zum Einschieben in den zweiten Behälterteil 4" ausgebildet, wozu dessen Außenmaße an in Innenmaße des zweiten Behälterteils 4" angepasst sind.

An dem Boden des zweiten Behälterteils 4" sind insgesamt vier Sicherungsstifte 10 (von denen nicht alle zu sehen sind) vorgesehen, die einstückig ausgebildet und somit unlösbar vom zweiten Behälterteil 4" sind. Einer der Sicherungsstifte 10 ist im Detail in der Schnittansicht gemäß Figur 4', 4" gezeigt. Der Sicherungsstift 10 weist einen aus der Bewandung 5 des zweiten Behälterteils 4", nämlich dessen Boden, hervorstehenden Schaft 11 auf, an dessen Kopfende ein Rastelement 12 ausgebildet ist.

Am ersten Behälterteil 4' sind Durchstecköffnungen 20 vorgesehen, durch die die Sicherungsstifte 10 des zweiten Behälterteils 4" geführt werden und einrasten können. Dabei sind die Durchstecköffnungen 20 separat von den Lochblechöffnungen vorgesehen und in ausreichender Zahl und Anordnung vorhanden, um die beiden Behälterteile 4', 4" zumindest in den in Figuren 1b und 1c dargestellten relativen Positionen sichern zu können. Zum Zusammensetzen der beiden Behälterteile 4', 4" wird der zweite Behälterteil 4" in der durch den Pfeil 90 angedeuteten Richtung so in den ersten Behälterteil 4' eingeschoben, dass die Sicherungsstifte 10 in die gewünschten Durchstecköffnungen 20 eingeführt werden und dort einrasten. Die Rastelemente 12 sind so ausgebildet, dass sie durch Aufbringen einer vorgegebenen Losbrechkraft entgegen der Richtung 90 werkzeugfrei gelöst werden können.

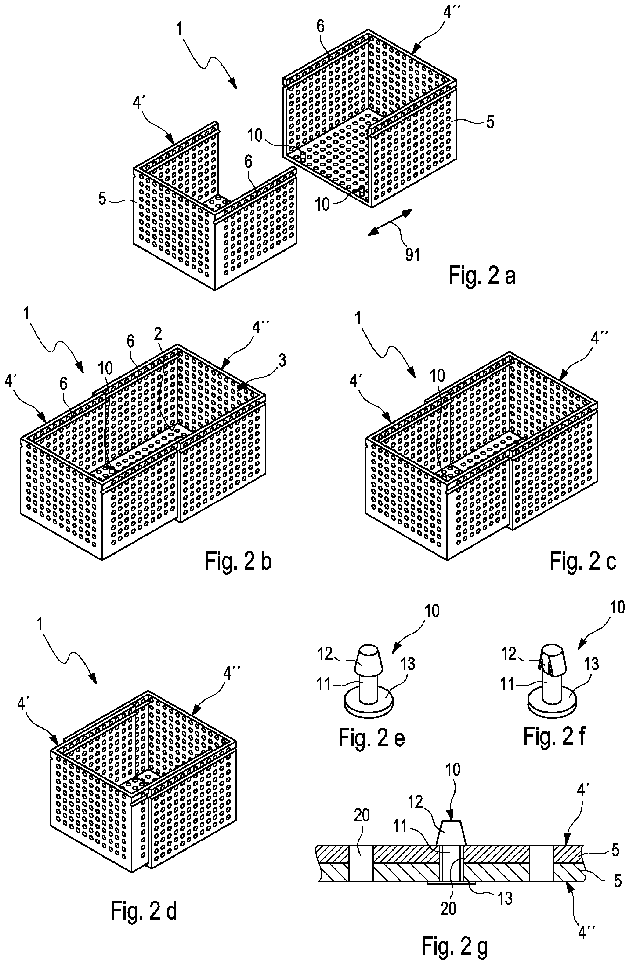

In Figur 2 ist ein zweites Ausführungsbeispiel eines erfindungsgemäßen Siebkorbs 1 für die Aufbereitung von medizinischen Instrumenten gezeigt. Figur 2a zeigt die beiden Behälterteile 4', 4" des Siebkorbs 1 in separiertem Zustand, während die Figuren 2b, 2c und 2d den Siebkorb 1 in unterschiedlichen Größenkonfigurationen zeigt. Figuren 2e und 2f zeigen zwei Beispiele möglicher Sicherungsstifte 1 gemäß dem zweiten Ausführungsbeispiel, deren Verwendung in Figur 2g skizziert ist.

Wie bereits beim Ausführungsbeispiel gemäß Figur 1, weist der Siebkorb 1 für die Aufbereitung von medizinischen Instrumenten im ordnungsgemäß zusammengesetzten Zustand der beiden Behälterteile 4', 4" eine rechteckige Grundfläche 2 und eine offene Oberseite 3 auf. Die beiden, den Siebkorb 1 bildendenden Behälterteile 4', 4" weisen eine siebartige Bewandung 5 aus Edelstahl-Lochblech auf, und sind an jeweils einer Seite offen. Außerdem weisen die beiden Behälterteile 4', 4" noch eine Führung 6 auf, die ein Einschieben des ersten Behälterteils 4' in den zweiten Behälterteil 4" ausschließlich in Richtung des Pfeils 91 gestattet. Hierzu sind selbstverständlich wieder Außenmaße des ersten Behälterteils 4' an in Innenmaße des zweiten Behälterteils 4" angepasst.

Zur Sicherung der beiden Behälterteile 4', 4" zueinander in einer der in Figuren 2b, 2c und 2d gezeigten Positionen sind zwei Sicherungsstifte 10 vorgesehen, die von den Behälterteilen 4', 4" separat ausgebildet sind. Figuren 2e und 2f zeigen zwei alternative Ausgestaltungen entsprechender Sicherungsstifte 10. Beide Sicherungsstifte 10 umfassen einen Schaft 11 mit einem Rastelement 12 am Kopfende. Am Fußende ist jeweils ein Halteelement 13 vorgesehen, welches ein Durchrutschen des Sicherungsstifts 10 durch eine Durchstecköffnung 20 verhindert. Der Sicherungsstifte 10 gemäß Figur 2a ist aus elastischem Material gefertigt, sodass das Rastelement 12 manuell so zusammengedrückt werden kann, dass es zum Lösen des Sicherungsstifts 10 durch eine auf den Schaft 11 angepasste Durchstecköffnung 20 geführt werden kann. Der Sicherungsstift 10 gemäß Figur 2f ist aus Edelstahl gefertigt, wobei das Rastelement 11 zwei Rastzungen aufweist, die aber ebenfalls zum Lösen des Sicherungsstifts 10 ausreichend weit werkzeugfrei zusammengedrückt werden können.

In Figur 2g ist die Verwendung eines Sicherungsstiftes 10 gemäß einer der Figuren 2e und 2f skizziert. Die beiden Behälterteile 4', 4" sind in Richtung 91 so ineinandergeschoben, dass die Öffnungen der Bewandung 5 aus Lochblech, die gleichzeitig Durchstecköffnungen 20 darstellen, miteinander fluchten. Der Sicherungsstift 10 wird dann durch die Durchstecköffnungen 20 in beiden Behälterteilen 4', 4" gesteckt. Zum Lösen wird das Rastelement 12 werkzeugfrei zusammengedrückt und aus den Durchstecköffnungen 20 herausgezogen.

## Patentansprüche

1. Siebkorb (1) für die Aufbereitung von medizinischen Instrumenten mit im wesentlichen rechteckiger Grundfläche (2) und offener Oberseite (3), umfassend einen ersten und einen zweiten Behälterteil (4', 4"), die an jeweils einer Stirnseite offen sind und eine siebartige Bewandung (5) aufweisen, wobei der erste Behälterteil (4') zum Einschieben in den zweiten Behälterteil (4") ausgebildet ist und wenigstens ein durch geeignete Durchstecköffnungen (20) hindurchsteckbaren Sicherungsstift (10) zur wahlweisen Sicherung der ineinandergeschobenen Behälterteile (4', 4") in wenigstens zwei unterschiedlichen Positionen zueinander vorgesehen ist.

2. Siebkorb nach Anspruch 1,
**dadurch gekennzeichnet, dass**
der wenigstens eine Sicherungsstift (10) unlösbar an einem Behälterteil (4") als aus dessen Bewandung (5) hervorstehender Schaft (11) mit Rastelement (12) am Kopfende ausgebildet ist und der andere Behälterteil (4') wenigstens zwei Durchstecköffnungen (20) aufweist.

3. Siebkorb nach Anspruch 1,
**dadurch gekennzeichnet, dass**
der wenigstens eine Sicherungsstift (10) einen Schaft (11) mit einem Halteelement (13) am Fußende und einem Rastelement (12) am Kopfende zum Durchstecken durch fluchtende Durchstecköffnungen (20) in beiden Behälterteilen (4', 4") ausgebildet ist.

4. Siebkorb nach Anspruch 3,
**dadurch gekennzeichnet, dass**
eine Führung (6) zwischen den beiden Behälterteilen (4', 4") vorgesehen ist, welche eine relative Bewegung der ineinandergeschobenen Behälterteile (4', 4") senkrecht zur offenen Oberseite (3) verhindert.

5. Siebkorb nach einem der vorhergehenden Ansprüche,
**dadurch gekennzeichnet, dass**
wenigstens ein Behälterteil wenigstens ein Positionierungselemente aufweist, welches ein Sichern der beiden Behälterteil (4', 4") nur in solchen Positionen zulässt, in denen die Durchlöcherung der Bewandung (5) der beiden Behälterteil (4', 4") im Überlappungsbereich wenigstens weitestgehend totraumfrei ist.

6. Siebkorb nach einem der vorhergehenden Ansprüche,
**dadurch gekennzeichnet, dass**
das Rastelement (12) des wenigstens einen Sicherungsstiftes (10) werkzeugfrei lösbar ist.

7. Siebkorb nach einem der vorhergehenden Ansprüche,
**dadurch gekennzeichnet, dass**
die Durchstecköffnungen (20) gesondert von einer siebartigen Durchlöcherung der Bewandung (5) oder Bestandteil der Durchlöcherung der Bewandung (5) sind.

8. Siebkorb nach einem der vorhergehenden Ansprüche,
**dadurch gekennzeichnet, dass**
wenigstens zwei, vorzugsweise wenigstens vier Sicherungsstifte (10) vorgesehen sind.

9. Siebkorb nach einem der vorhergehenden Ansprüche,
**dadurch gekennzeichnet, dass**
die Behälterteile (4', 4") wenigstens teilweise aus Lochblech, Drahtgeflecht oder geschweißtem Drahtgitter sind.

10. Siebkorb nach einem der vorhergehenden Ansprüche,
**dadurch gekennzeichnet, dass**
die Behälterteile (4', 4") und/oder der wenigstens eine Sicherungsstift (10) aus Edelstahl sind.

11. Deckel für einen Siebkorb, vorzugsweise nach einem der vorhergehenden Ansprüche, umfassend einen ersten und einen zweiten Deckelteil, die in wenigstens zwei verschiedenen Position zueinander anordnenbar sind und wenigstens ein durch geeignete Durchstecköffnungen hindurchsteckbaren Sicherungsstift (10) zur wahlweisen Sicherung der Deckelteile in wenigstens zwei unterschiedlichen Positionen zueinander vorgesehen ist.

12. Deckel nach Anspruch 11,
**dadurch gekennzeichnet, dass**
die beiden Deckelteile eine Führung aufweisen, entlang derer sich die beiden Deckelteile relativ zueinander verschieben lassen.

13. Deckel nach Anspruch 11 oder 12,
**dadurch gekennzeichnet, dass**
der wenigstens eine Sicherungsstift (10) einen Schaft (11) mit einem Halteelement (13) am Fußende und einem Rastelement (12) am Kopfende zum Durchstecken durch fluchtende Durchstecköffnungen (20) in beiden Deckelteilen ausgebildet ist.

14. Deckel nach Anspruch 11,
**dadurch gekennzeichnet, dass**
der wenigstens eine Sicherungsstift (10) unlösbar an einem Deckelteil als aus dessen Bewandung (5) hervorstehender Schaft (11) mit Rastelement (12) am Kopfende ausgebildet ist und der andere Deckelteil wenigstens zwei Durchstecköffnungen (20) aufweist.

15. Deckel nach einem der Ansprüche 11 bis 14,
**dadurch gekennzeichnet, dass**
- das Rastelement (12) des wenigstens einen Sicherungsstiftes (10) werkzeugfrei lösbar ist;
- die Durchstecköffnungen (20) gesondert von einer siebartigen Durchlöcherung der Deckelteile oder Bestandteil der Durchlöcherung der Deckelteile sind;
- wenigstens zwei, vorzugsweise wenigstens vier Sicherungsstifte (10) vorgesehen sind;
- die Deckelteile wenigstens teilweise aus Lochblech, Drahtgeflecht oder geschweißtem Drahtgitter sind; und/oder
- die Deckelteile und/oder der wenigstens eine Sicherungsstift (10) aus Edelstahl sind.
